# FASCICULE DE BREVET EUROPEEN

(11) **EP 0 320 419 B1**
(45) Date de publication et mention de la délivrance du brevet: **10.03.1993**
(21) Numéro de dépôt: 88420414.0
(22) Date de dépôt: 09.12.1988
(51) Int. Cl.: A61M 1/16, B01D 61/00

(54) **Procédé et dispositif de rinçage et d'amorçage d'un échangeur**
Verfahren und Vorrichtung zum Spülen und Starten einer Austauschvorrichtung
Process and apparatus for rinsing and priming an exchange device

(30) Priorité: 11.12.1987 FR 8717608
(43) Date de publication de la demande: 14.06.1989
(73) Titulaire: HOSPAL INDUSTRIE, F-69330 Meyzieu (FR)
(72) Inventeur: Richalley, Gérard, F-69006 Lyon (FR); Burtin, Jacques, F-69320 Feyzin (FR)

(56) Documents cités:
- EP-A- 0 161 686
- EP-A- 0 181 139
- WO-A-85/03879
- US-A- 3 061 518
- J. BIOMED. MATER. RES., vol. 8, 1974, pages 163-183, John Wiley & Sons Inc.; R.C. DUTTON et al.: "Formation of platelet aggregate emboli in a prototype hollow fiber membrane oxygenator"

## Description

La présente invention concerne le domaine technique du rinçage et de l'amorçage d'un dispositif destiné au traitement extracorporel du sang.
Plus particulièrement, la présente invention a pour objet un procédé de rinçage et d'amorçage d'un échangeur à deux compartiments séparés par une membrane semi-perméable, le premier compartiment étant destiné à la circulation extracorporelle du sang à traiter et le second compartiment étant relié à un circuit de liquide de dialyse. Bien que les dispositifs de traitement extracorporel du sang fassent l'objet des plus grands soins durant toute leur élaboration et que la phase finale du processus de fabrication soit une phase de stérilisation, il est nécessaire de rincer correctement les échangeurs avant leur utilisation. Ce rinçage a pour but d'éliminer les éventuels résidus de fabrication et de stérilisation, qui pourraient entrainer, en cas de passage dans la circulation sanguine du patient, des réactions tout à fait indésirables, telles que les réactions dites d'hypersensibilité.
Cette phase de rinçage est habituellement effectuée en faisant circuler dans le premier compartiment ou compartiment sang de l'hémodialyseur du liquide physiologique stérile provenant d'une poche-réservoir prévue à cet effet et en faisant circuler dans le second compartiment ou compartiment dialysat du liquide fabriqué par un générateur de liquide de dialyse généralement intégré au moniteur de dialyse. Le liquide stérile destiné au rinçage du compartiment sang est généralement mis en circulation grâce à une pompe habituellement utilisée pour la circulation du sang à traiter.

Afin d'assurer un rinçage efficace du compartiment sang de l'hémodialyseur, la demande de brevet français publiée sous le no 2 566 273 propose notamment une méthode de lavage selon laquelle :
- on fait tout d'abord circuler dans le compartiment sang une quantité prédéterminée d'une solution saline physiologique, par exemple une solution aqueuse à 0,9% de chlorure de sodium que l'on évacue immédiatement après son passage,
- on fait ensuite recirculer dans le compartiment sang, pendant une durée prédéterminée une certaine quantité de solution saline physiologique fraiche,
- enfin, on effectue un rinçage final grâce à une solution fraiche provenant d'une seconde source de solution saline physiologique.

Un tel procédé de rinçage, s'il présente l'avantage d'éliminer efficacement les résidus éventuellement présents dans le compartiment sang de l'hémodialyseur, représente cependant une sujetion importante pour le personnel hospitalier chargé de la préparation de la séance d'hémodialyse.
En outre, le procédé proposé ne permet pas de résoudre les problèmes pouvant survenir en raison de l'utilisation pour le rinçage du compartiment-dialysat, de liquide de dialyse éventuellement contaminé. En effet, il est possible que des substances telles que des dérivés bactériens, diffusent à travers la membrane semi-perméable du compartiment dialysat vers le compartiment sang.
De plus, au moment du branchement du compartiment sang de l'hémodialyseur avec la source de sang à traiter, il est très difficile de contrôler efficacement les pressions s'exerçant à l'intérieur de chacun des compartiments de l'hémodialyseur, et donc d'éviter que des substances éventuellement présentes dans le liquide de dialyse traversent la membrane. Le problème est d'autant plus important que la membrane utilisée est plus perméable.
Dans le cas où le sang ainsi contaminé est ensuite renvoyé au patient, cela pourrait entrainer des réactions dites d'hypersensibilité se développant dès les premières minutes de la séance d'hémodialyse et pouvant conduire le médecin à interrompre immédiatement la séance.

Afin d'améliorer la qualité bactériologique du liquide de dialyse préparé, l'état de la technique a proposé notamment de filtrer le liquide de dialyse avant son utilisation. Une telle solution nécessite l'utilisation de filtres, ce qui représente des opérations d'entretien et une augmentation du coût du générateur de liquide de dialyse. En outre, l'efficacité d'un tel système est, à ce jour, tout à fait moyenne. En effet, s'il est effectivement probable que toutes les bactéries sont retenues par le filtre utilisé, ce n'est certainement pas le cas de tous les dérivés bactériens pouvant produire un effet biologique et dont la taille est si faible qu'il est difficile de pouvoir les retenir par filtration. Un tel système suppose en outre des moyens pour vérifier en ligne l'efficacité de la filtration ainsi qu'un dispositif de sécurité permettant d'éviter l'utilisation d'un liquide de dialyse pour lequel l'efficacité de la filtration s avérerait insuffisante.

Selon le document WO-A-85/03 879, il est connu un procédé de rinçage et d'amorçage d'une unité d'ultrafiltration constituée par exemple par un dialyseur dans lequel le compartiment dialysat devient le compartiment ultrafiltrat. Il n'y a donc en principe pas, dans ce cas, de circulation de liquide de dialyse. Le procédé divulgué par ce document consiste à introduire la solution de rinçage à l'entrée du compartiment sang et à appliquer une dépression à la sortie ultrafiltrat afin d'aspirer la solution de rinçage à travers le compartiment sang ainsi que, du moins partiellement, à travers la membrane. Une telle méthode n'est cependant pas appropriée pour rincer et dégazer correctement le second compartiment d'un échangeur destiné à être parcouru ensuite par un liquide épurateur préparé par un générateur de liquide de dialyse.

Il n'existe donc pas actuellement, dans l'art antérieur, de procédé satisfaisant de rinçage et d'amorçage d'un échangeur.

L'objet de la présente invention est donc de proposer un procédé de rinçage et d'amorçage d'un échangeur ne présentant pas les inconvénients de l'art antérieur et permettant d'éliminer efficacement les éventuels résidus de fabrication et de stérilisation.

Un autre objet de la présente invention est de proposer un procédé de rinçage et d'amorçage d'un échangeur permettant de s'affranchir le plus possible des contaminations du liquide fabriqué par le générateur de liquide de dialyse.

Un autre objet de la présente invention est de proposer un procédé de rinçage et d'amorçage d'un hémodialyseur de mise en oeuvre simple et rapide pour le personnel hospitalier chargé de la préparation de la séance d'hémodialyse.

Un autre objet de la présente invention est de proposer un procédé de rinçage et d'amorçage d'un hémodialyseur dont l'efficacité permet d'éviter qu'une réaction d'hypersensibilité ne se produise au début de la séance de traitement.

Un autre objet de la présente invention est de proposer un procédé de rinçage et d'amorçage d'un échangeur n'entrainant pas d'augmentation notable du coût de la séance de traitement.

Un autre objet de la présente invention est de proposer un procédé de rinçage et d'amorçage convenant particulièrement bien pour un échangeur équipé d'une membrane présentant une perméabilité à l'eau élevée.

Un autre objet de la présente invention est de proposer un procédé de rinçage et d'amorçage d'un échangeur ne nécessitant pas l'utilisation du générateur de liquide de dialyse.

Pour atteindre ces différents buts, la présente invention propose un procédé de rinçage et d'amorçage d'un échangeur du type comprenant une membrane semi-perméable à deux faces, la première face délimitant un premier compartiment destiné à la circulation du sang à traiter, la seconde face délimitant un second compartiment destiné à la circulation d'un liquide épurateur préparé par un générateur de liquide de dialyse, procédé selon lequel on fait circuler dans le premier compartiment une solution stérile, et on fait circuler dans le second compartiment une solution stérile, caractérisé en ce que :
- on fait circuler la solution stérile dans le premier compartiment et la solution stérile dans le second compartiment à co-courant,
- on effectue un dépôt de protéines sur au moins une face de ladite membrane semi-perméable.

Selon un mode de réalisation de la présente invention, on effectue le dépôt de protéines en faisant circuler dans le premier compartiment de l'échangeur, une solution stérile d'albumines, de préférence humaines.

Selon un autre mode de réalisation de la présente invention, on effectue le dépôt de protéines postérieurement au rinçage du premier compartiment, en faisant circuler le sang à traiter dans le premier compartiment, et en maintenant la solution stérile dans le second compartiment pendant la première phase de circulation du sang.

Selon un mode avantageux de réalisation de la présente invention, on fait circuler la solution saline physiologique stérile successivement dans le premier puis dans le second compartiment de l'hémodialyseur.

D'autres caractéristiques et avantages de la présente invention apparaitront au cours de la description qui va suivre en référence aux dessins annexés qui illustrent, à titre d'exemples, de façon schématique et sans échelle déterminée, différents modes de réalisation de la présente invention.

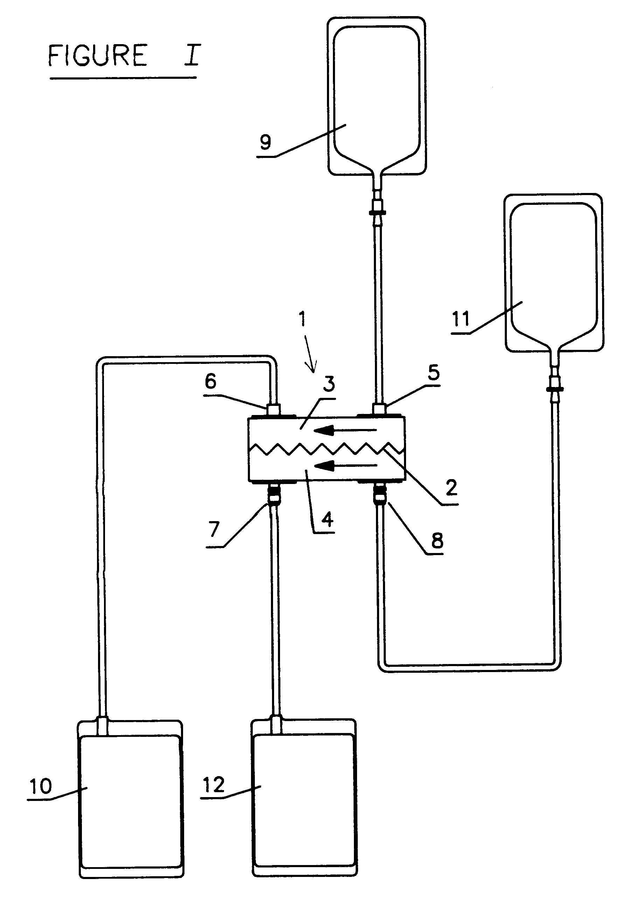

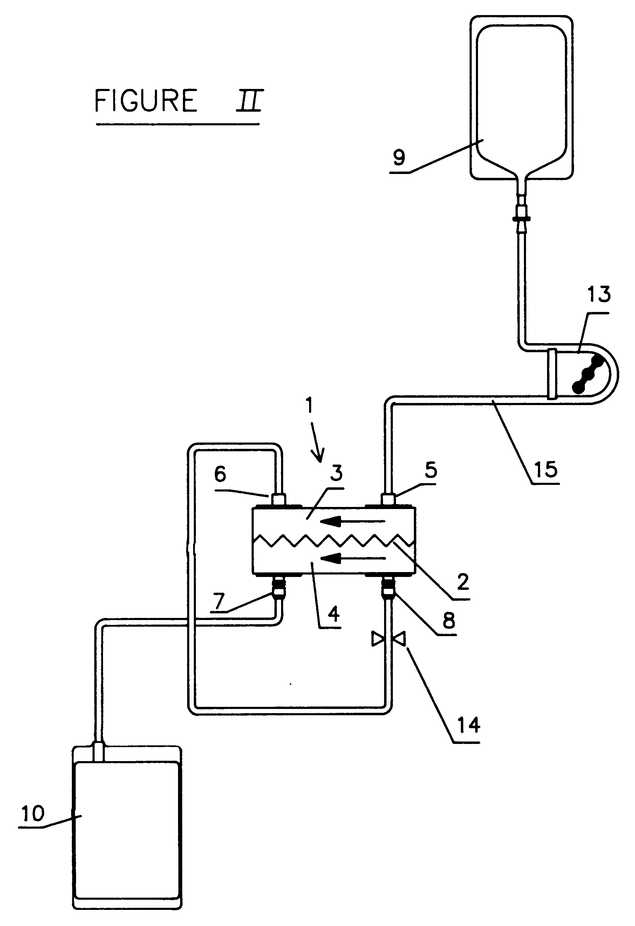

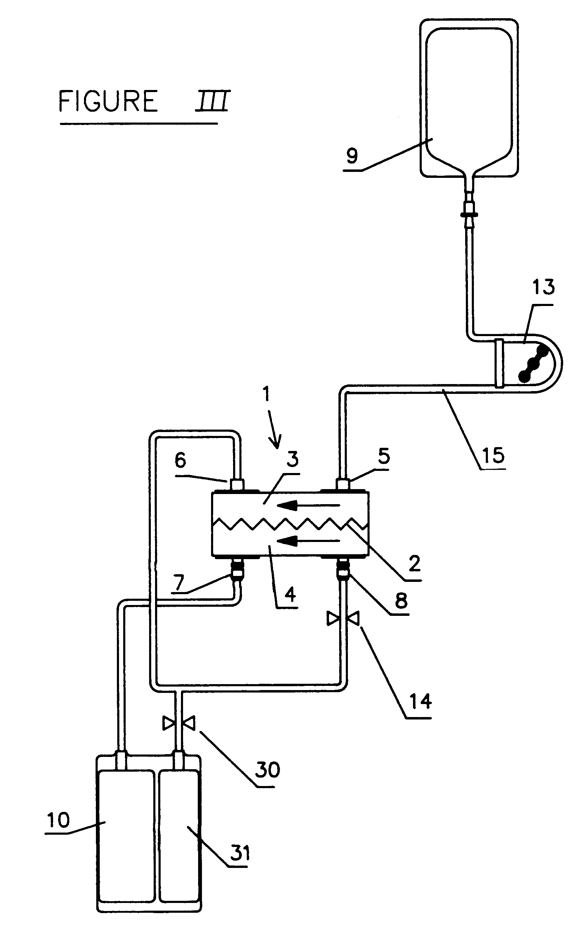

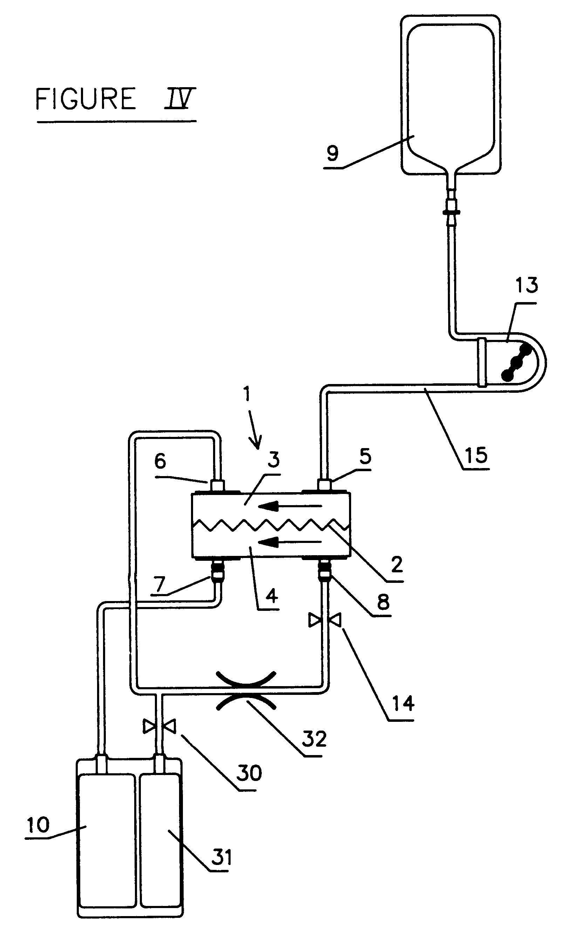

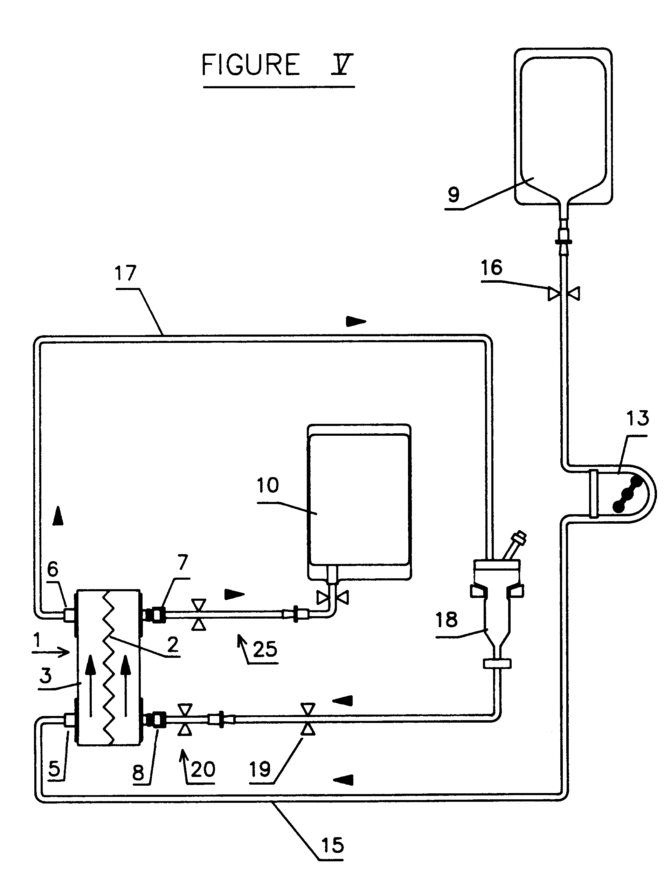

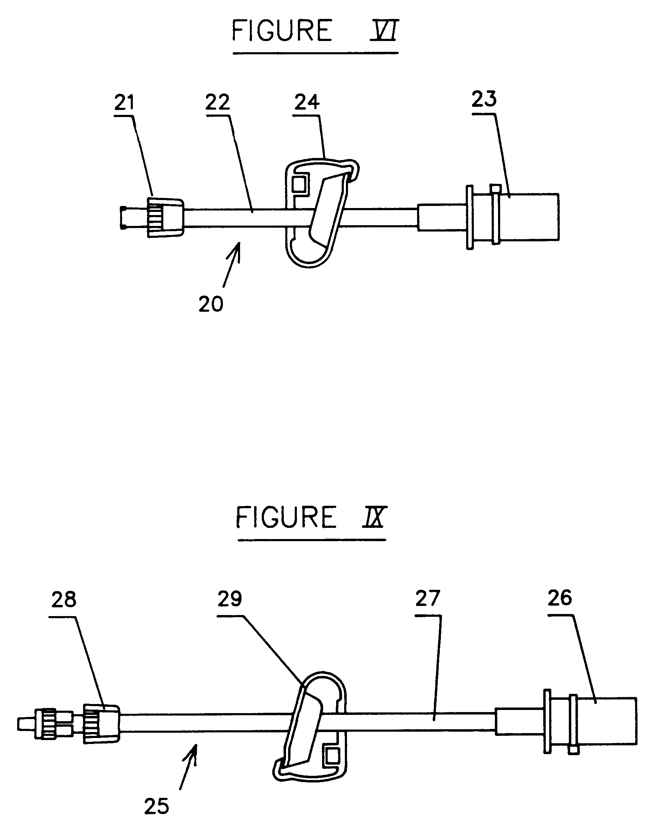

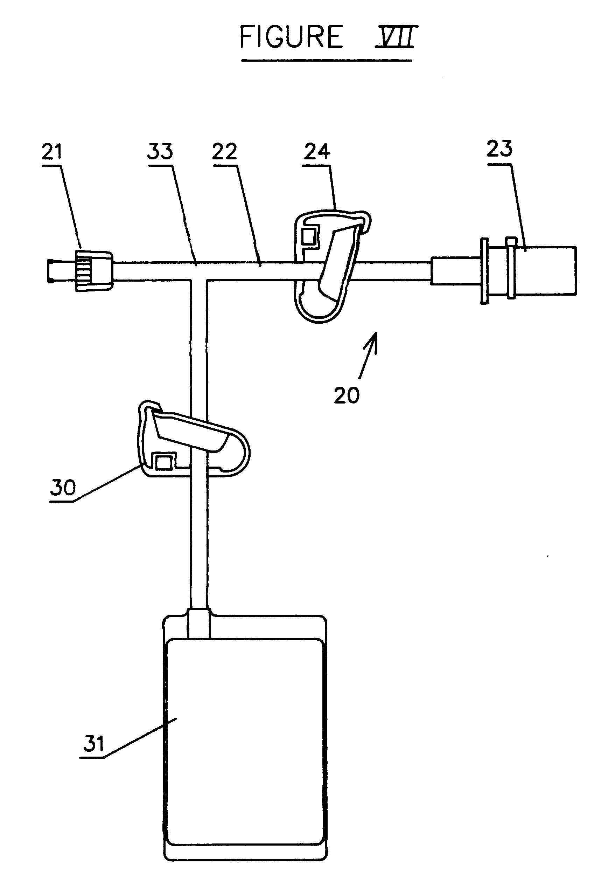

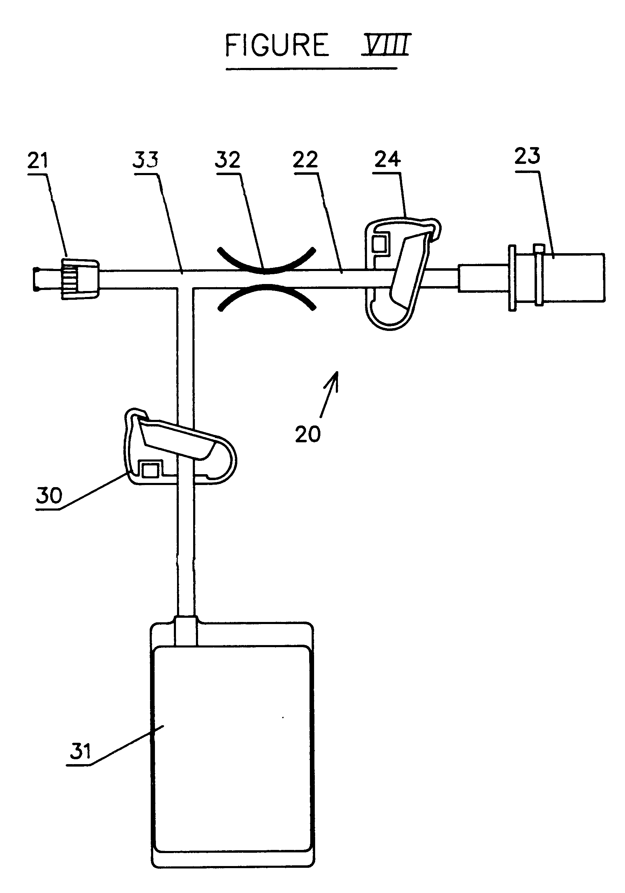

La figure I représente de façon schématique un premier mode de réalisation du procédé selon la présente invention.

La figure II représente de façon schématique un second mode de réalisation du procédé selon la présente invention.

La figure III représente de façon schématique un troisième mode de réalisation du procédé selon la présente invention.

La figure IV représente de façon schématique une variante de réalisation du mode de réalisation représenté à la figure III.

La figure V représente un mode de réalisation du dispositif de mise en oeuvre du procédé selon la présente invention.

La figure VI représente de façon schématique un premier mode de réalisation du segment de connexion 20 du dispositif représenté sur la figure V.

La figure VII représente de façon schématique un second mode de réalisation du segment de connexion 20.

La figure VIII représente de façon schématique un troisième mode de réalisation du segment de connexion 20.

La figure IX représente de façon schématique la ligne de recueil 25 du dispositif représenté sur la figure V.

Ainsi que cela apparait sur la figure I, on voit que le procédé selon la présente invention concerne le rinçage et l'amorçage d'un échangeur 1 comprenant une membrane 2 séparant deux compartiments 3 et 4, le premier compartiment 3 étant destiné à la circulation du sang à traiter, le second compartiment 4 étant destiné à la circulation d'un liquide épurateur préparé généralement par un générateur de liquide de dialyse (non représenté). L'échangeur 1 est, par exemple, un hémodialyseur ou un hémodiafiltre.

Le premier compartiment ou compartiment sang 3 comporte un orifice 5 destiné à l'entrée du sang à traiter et un orifice 6 destiné à la sortie du sang traité. De façon similaire, le second compartiment ou compartiment dialysat 4 comporte un orifice 7 d'entrée du liquide de dialyse et un orifice 8 de sortie du liquide de dialyse . Avantageusement, ces différents orifices sont constitués par des embouts de type connu en soi. Avant l'introduction du sang à traiter, on rince le compartiment sang 3 grâce à une solution de liquide stérile provenant d'une poche réservoir 9.
Cette solution stérile est avantageusement une solution saline d'albumines humaines, dont la concentration est, par exemple, de 4 à 10 g/l. Cette solution peut être mise en circulation, par simple action de la gravité en disposant la poche à une hauteur convenable.

Il est cependant préférable d'utiliser une pompe afin de pouvoir régler correctement le débit de circulation de cette solution.

Après son passage dans le compartiment 3, le liquide est évacué par l'orifice 6 et recueilli dans une poche 10 qui est de préférence placée de manière à permettre la circulation du liquide par simple gravité.

Le compartiment dialysat 4 est rincé en utilisant une solution stérile provenant d'une poche-réservoir 11. Cette solution stérile est avantageusement une solution saline physiologique, par exemple une solution de chlorure de sodium dont la concentration en poids est de 0,9 pourcent. Après son passage dans le compartiment 4, la solution usagée est évacuée vers la poche de recueil 12, qui est disposée avantageusement de manière à permettre la circulation par simple gravité de la solution provenant du compartiment 4. Il est en fait possible de prévoir, au lieu des deux poches de recueil 10 et 12, une poche unique dont la capacité est au moins égale à la somme des capacités des poches 9 et 11.
La solution stérile est mise en circulation à partir de la poche 11 par simple gravité en réglant convenablement la hauteur de la poche ou de façon avantageuse par l'action d'une pompe.

Dans le cas où la mise en circulation des solutions traversant respectivement les compartiments 3 et 4 de l'hémodialyseur est obtenue par le placement en charge des poches 9 et 11, la position relative des poches 9 et 11 doit être choisie convenablement, de manière à obtenir une pression du liquide à proximité de l'orifice 5 supérieure à la pression du liquide à proximité de l'orifice 8. La circulation des liquides dans chacun des compartiments s'effectue à co-courant, ce qui permet de maintenir une différence de pression sensiblement constante de part et d'autre de la membrane, tout au long de l'hémodialyseur, ce qui est une caractéristique fondamentale du procédé de la présente invention.
Ainsi, grâce à l'utilisation d'une solution d'albumines humaines pour rincer le compartiment sang de l'hémodialyseur, on obtient sur la face de la membrane en contact avec le compartiment sang un dépôt protéique constituant un film protecteur. On choisit la concentration en albumines de la solution en fonction de la nature de la membrane, et donc de son affinité pour les protéines, pour permettre le dépôt d'une couche protéique au moins monomoléculaire. Ce film protecteur entraine une amélioration très sensible de la biocompatibilité de la membrane ; il permet également de constituer une barrière à toutes les substances pouvant provenir du liquide présent dans le compartiment-dialysat.
Afin de favoriser la formation de ce dépôt protéique, on peut provoquer une différence de pression du part et d'autre de la membrane ; ainsi, si la pression du liquide dans le compartiment sang 3 est plus importante que la pression du liquide dans le compartiment dialysat 4, le passage par convection du liquide du compartiment sang vers le compartiment dialysat favorisera le dépôt des protéines sur la membrane.
Après que le compartiment sang ait été convenablement rincé avec la solution d'albumines, l'orifice 5 du compartiment 3 est relié à une source de sang à traiter, par exemple au système artériel d'un patient alors que l'orifice 6 est relié à un système de recueil du sang traité, par exemple au système veineux du patient. Il est alors possible de régler la différence de pression de part et d'autre de la membrane 2 de manière à provoquer un passage de liquide par convection du compartiment sang vers le compartiment dialysat. Ce liquide est habituellement appelé ultrafiltrat. Ensuite, on peut relier les orifices 7 et 8 du compartiment 4 respectivement à une source de liquide de dialyse et à des moyens d'évacuation de ce liquide de dialyse. La séance de traitement du sang peut alors être effectuée, en réglant les différents paramètres de façon habituelle.
Grâce au dépôt du film protéique sur la face de la membrane en contact avec le compartiment sang, dans le cas où, pour une raison quelconque, le sens de la différence de pression de part et d'autre de la membrane s'inverserait, les substances contaminantes éventuellement présentes dans le liquide de dialyse ne parviendront pas jusqu'aù sang ; en effet, même si elles réussissent à traverser la membrane 2, on a observé que tout se passe comme si elles étaient arrêtées par le film protéique protégeant la face en contact avec le compartiment sang.

Cette barrière protéique permet également d'éviter le passage par diffusion des substances contaminantes du compartiment dialysat vers le compartiment sang.

Selon un autre mode de réalisation du procédé selon la présente invention, on peut également rincer le compartiment sang 3 avec une solution saline physiologique stérile ; après cette phase de rinçage, et avant de faire circuler le sang à traiter, on fait passer dans le compartiment sang 3 une solution stérile d'albumines humaines dont la concentration permet d'obtenir très rapidement une couche au moins monomoléculaire de protéines sur la membrane. On peut, par exemple faire circuler 1l d'une solution à 40 g/l d'albumines avec un débit de 100 ml/mn.
Dans ce cas, il est alors possible de prévoir une source unique de solution stérile pour le rinçage des deux compartiments de l'hémodialyseur, au lieu des deux poches 9 et 11.

Selon un autre mode de réalisation du procédé selon la présente invention, on fait circuler dans le compartiment sang 3 une solution saline physiologique stérile et on fait circuler dans le compartiment dialysat 4, une solution d'albumines stérile. Dans ce cas, le dépôt de protéines est réalisé, non pas sur la face de la membrane délimitant le compartiment sang, mais sur la face de la membrane délimitant le compartiment dialysat.

Selon un autre mode de réalisation de la présente invention, on fait circuler une solution stérile d'albumines dans chacun des compartiments 3 et 4 de l'échangeur. Ainsi, on effectue un dépôt de protéines sur chacune des faces de la membrane.

Selon un mode de réalisation avantageux du procédé selon la présente invention, on réalise le dépôt sur la membrane d'une couche protéique non pas à partir d'une solution stérile d'albumines humaines, mais directement grâce aux protéines présentes dans le sang à traiter. Ceci présente l'avantage de diminuer notablement le coût du procédé.
Dans ce cas, on rince les compartiments 3 et 4 de l'hémodialyseur en faisant circuler à co-courant dans chacun d'eux une solution stérile provenant des deux poches 9 et 11 ainsi que cela est représenté sur la figure I, ou d'une source unique.
Lorsque le rinçage du compartiment sang est suffisant, c'est-à-dire par exemple après 10 mn de circulation de la solution avec un débit de 200 ml/mn on relie l'orifice d'entrée du sang 5 avec la source de sang à traiter, qui circule alors à l'intérieur du compartiment 3. Lorsque toute la solution de rinçage a quitté le compartiment 3, poussée par le sang, on interrompt un instant la circulation et on relie l'orifice de sortie du sang 6 aux moyens de recueil (non représentés) du sang traité. Pendant cette première phase de circulation du sang dans l'hémodialyseur, on maintient la solution de rinçage stérile présente dans le compartiment dialysat 4, cette solution étant maintenue en circulation ou pas.
On peut également appliquer dans le compartiment dialysat une dépression, de façon à provoquer un passage d'ultrafiltrat par convection du compartiment sang vers le compartiment dialysat.

Ainsi, durant cette première phase de circulation du sang, les protéines sanguines se déposent sur la membrane et forment un film protecteur. Ce dépôt est d'autant plus rapide que l'affinité de la membrane pour les protéines est grande. Par exemple, la membrane AN 69 (marque déposée) commercialisée par la société HOSPAL est une membrane qui présente une très grande affinité pour les protéines. Des essais ont montré qu'une circulation du sang à traiter pendant 3 à 5mn avec un débit de 100 à 200 ml/mn permet d'obtenir un dépôt protéique suffisant pour constituer un film protecteur.

On a observé que le maintien de la solution de rinçage stérile dans le compartiment dialysat 4 permet de s'affranchir des risques de contamination du sang par des substances éventuellement présentes dans le liquide de dialyse, fourni normalement par un générateur de liquide de dialyse, dans le cas d'un passage par diffusion à travers la membrane ainsi que dans le cas où, pour une raison quelconque, la pression du liquide dans le compartiment dialysat 4 deviendrait supérieure à la pression du sang dans le compartiment 3.

Après que cette première phase de circulation du sang ait permis de constituer un dépôt protéique suffisant, la solution de rinçage stérile est remplacée dans le compartiment 4 par le liquide épurateur préparé par le générateur de liquide de dialyse.

La séance se poursuit alors de façon habituelle en fixant de façon appropriée les différents paramètres pour obtenir le traitement souhaité du sang.

Selon le mode de réalisation représenté sur la figure II, on effectue le rinçage des deux compartiments 3 et 4 de l'hémodialyseur 1 en série avec la même solution stérile provenant de la poche réservoir 9. On utilise de préférence une poche de 21 d'une solution stérile de chlorure de sodium à 0,9 pourcent.

Cette solution est, avantageusement mise en circulation grâce à une pompe 13 ; on peut, par exemple utiliser la pompe artérielle de l'appareil de dialyse. Le débit fourni par cette pompe est par exemple réglé à 100 ml/mn jusqu'à ce que le compartiment 3 soit rempli, on peut ensuite continuer le rinçage avec un débit de 200 ml/mn.
Le liquide de rinçage traverse successivement le compartiment sang 3 puis le compartiment dialysat 4 et est recueilli ensuite dans la poche 10. La circulation du liquide dans chacun des compartiments s'effectue avantageusement à co-courant, de façon à maintenir sensiblement la même différence de pression entre le compartiment sang et le compartiment dialysat sur toute l'étendue de la membrane.

Lorsque la poche 9 est vide, on arrête le fonctionnement de la pompe 13 ; on remplace alors la poche 9 par la source de sang à traiter, par exemple en reliant la ligne artérielle 15 au système artériel d'un patient.
On remet la pompe 13 en route pour faire circuler le sang à traiter avec un débit par exemple de 100 ml/mn.
Le sang à traiter pénètre alors à l'intérieur de l'hémodialyseur. Lorsque le compartiment sang 3 est rempli, on relie l'orifice de sortie sang 6 à des moyens de recueil du sang traité, par exemple au système veineux d'un patient.
Pendant ce temps, la circulation de la solution stérile à l'intérieur du compartiment dialysat 4 est arrêtée, grâce par exemple à la fermeture d'un clamp 14, ou tout autre dispositif d'obturation connu en soi.
On fait ensuite circuler le sang à traiter à l'intérieur de l'hémodialyseur pendant 3 à 5mn, de façon à permettre sur la membrane 2 un dépôt de protéines suffisant pour former un film protecteur. Après cette première phase de circulation du sang à traiter, on remplace la poche de recueil 10 par une source de liquide de dialyse et on connecte l'orifice 8 à des moyens d'évacuation du liquide de dialyse usagé.
La séance de traitement du sang est alors effectuée de façon habituelle.

Des essais ont été effectués chez des patients présentant habituellement des réactions dites d'hypersensibilité de type anaphylactiques. Lors de la mise en oeuvre de ce procédé de rinçage et d'amorçage de l'échangeur, aucune réaction de ce genre ne s'est produite. Par contre, un retour aux méthodes de rinçage et d'amorçage habituelles a provoqué à nouveau les mêmes réactions d'hypersensibilité.

Au lieu d'utiliser comme solution de rinçage une solution stérile de chlorure de sodium à 0,9 pour cent, il est particulièrement avantageux d'utiliser une solution contenant en outre de l'héparine, par exemple une solution à 2000 UI/l d'héparine.

Ainsi, grâce à l'utilisation d'une solution héparinée, la membrane est imprégnée d'héparine pendant la phase de rinçage, ce qui est très important en ce qui concerne les problèmes de coagulation survenant principalement pendant les premières minutes de circulation extracorporelle du sang, lorsque les mesures de prévention de la coagulation ne sont pas correctement respectées.

En effet, grâce à l'utilisation d'une telle solution de rinçage héparinée, le sang qui pénètre dans l'échangeur 1 est directement en contact avec une membrane imprégnée d'héparine et indirectement avec un liquide hépariné présent dans le compartiment dialysat 4. Ceci permet avantageusement de réduire les risques de coagulation.

Il est encore possible d'améliorer la qualité du rinçage en procédant selon la technique décrite ci-dessous en relation avec la figure III.

En effet, on sait que la plupart des résidus de fabrication sont éliminés dès la première phase de rinçage de l'hémodialyseur c'est à dire lors du passage de la première fraction du liquide de rinçage, de 500 ml par exemple. Il apparait donc avantageux de ne pas utiliser cette première fraction du liquide de rinçage provenant du compartiment 3 pour rincer le compartiment 4.

A cette fin, il est possible, dans une première phase d'ouvrir le clamp 30 et de fermer le clamp 14. La première fraction du liquide de rinçage, par exemple 500 ml est recueillie dans la poche 31. Quand la poche 31 est remplie, on arrête la pompe 13 pour fermer le champ 30 et ouvrir le clamp 14.
Puis, on remet la pompe 13 en route afin que dans une deuxième phase, le liquide de rinçage restant dans la poche 9 traverse successivement le compartiment 3 puis le compartiment 4 ainsi que cela a été décrit ci-dessus en relation avec la figure II.
L'arrêt de la pompe de circulation 13 lors de la manipulation d'ouverture et de fermeture des champs 30 et 14, est effectué par sécurité pour éviter de créer des surpressions dans le circuit.

La combinaison des deux champs 30 et 14 peut être remplacée par tout moyen équivalent, tel un robinet 3 voies permettant de mettre en communication la sortie 8 du compartiment 3, sélectivement soit avec la poche de recueil 31 soit, avec la sortie 8 du compartiment 2.

La solution technique préférentielle retenue pour recueillir la première fraction du liquide de rinçage dans la poche 31 est celle représentée sur la figure IV.
Selon ce mode de réalisation, durant la première phase du rinçage, le champ 30 est ouvert ; la présence d'une résistance hydraulique 32 sur la ligne de connexion entre la sortie 6 du compartiment 3 et la sortie 8 du compartiment 4, en aval de la communication possible avec la poche 31 crée un passage préférentiel du compartiment 3 vers la poche de recueil 31.

Lorsque la poche de recueil 31 est remplie, la pression monte dans la ligne de connexion, le liquide de rinçage s'écoule alors à travers la résistance hydraulique 32, le compartiment 4 avant d'être recueilli dans la poche 10. Par sécurité, on ferme le champ 30 lorsque la poche 31 est remplie.

La présence de la résistance hydraulique 32 présente l'avantage d'augmenter la différence de pression entre le compartiment 3 et le compartiment 4, favorisant ainsi le flux convectif dans le sens compartiment 3-compartiment 4. Cette augmentation du flux convectif permet à son tour de restreindre le passage par diffusion d'éléments indésirables du compartiment 4 vers le compartiment 3.

Afin de réaliser le procédé de rinçage et d'amorçage précédemment décrit, on utilise un dispositif de mise en oeuvre représenté plus en détails sur la figure V ; ce dispositif consiste en un ensemble comprenant :
- une ligne artérielle 15 reliant l'orifice d'entrée du sang 5 de l'hémodialyseur à la poche réservoir 9 ; cette ligne artérielle 15 comporte en amont de la pompe 13 et en aval de la poche réservoir 9 dans le sens de circulation de la solution stérile un dispositif d'obturation 16, par exemple un clamp,
- des moyens de communication entre l'orifice 6 de sortie du sang et l'orifice de sortie 8 du liquide de dialyse ; ces moyens de communication comportent avantageusement une ligne veineuse 17 traversant un piège à bulles 18 et comportant à son extrémité un dispositif d'obturation 19, tel un clamp, ainsi qu'un segment de connexion 20 entre l'extrémité de la ligne veineuse 17 et l'orifice 8 ; ce segment de connexion 20 est représenté de façon plus détaillée sur la figure VI ; il comporte, par exemple, un raccord du type Luer-Lock femelle 21 reliant l'extrémité de la ligne veineuse 17 à une ligne 22 avantageusement en chlorure de polyvinyle; cette ligne 22 comporte à l'extrémité opposée au raccord de type Luer-Lock 21, un connecteur 23 de type Hansen, capable de s'adapter à l'orifice 8 constituant la sortie dialysat. La ligne 22 comporte en outre un dispositif d'obturation 24 tel un clamp,
- une ligne de recueil 25 permettant de relier l'orifice 7 du compartiment dialysat 4 à la poche de recueil 10. Cette ligne de recueil, représenté à la figure IX, comporte par exemple un connecteur 26 reliant l'orifice 7 constituant l'entrée du compartiment dialysat à une ligne 27 avantageusement en chlorure de polyvinyle ; cette ligne 27 comporte à l'extrémité opposée au connecteur 26, un raccord de type Luer-Lock mâle 28 destiné à être relié au raccord de type Luer femelle correspondant de la poche de recueil 10. La ligne 27 comporte en outre un dispositif d'obturation 29 tel un clamp,

En vue de mettre en oeuvre le mode de réalisation représenté sur la figure III, le segment de connection 20 comporte ainsi que cela est représenté sur la figure VII une connexion en T 33 permettant au liquide de rinçage de s'écouler soit dans la poche de recueil 31 dans le cas où le clamp 30 est ouvert et le clamp 24 fermé, soit dans le compartiment 4 dans le cas inverse où le clamp 30 est fermé et le clamp 24 ouvert.
Le clamp 24 joue ici le rôle du clamp 14 décrit à la figure III.

La mise en oeuvre du mode de réalisation représenté sur la figure IV peut être obtenue grâce à la modification du segment de connexion 20 tel que représenté sur la figure VIII. Cette modification consiste essentiellement en l'adjonction d'une résistance hydraulique 32, en amont du connecteur Hansen 23 et en aval de la connexion en T 33. Cette résistance 32 peut par exemple prendre la forme d'un tube 22 dont une portion présente un diamètre interne réduit.

La poche de recueil 31 peut être une poche indépendante dont le volume est égal au volume de la première fraction du liquide de rinçage que l'on ne souhaite pas faire circuler dans le compartiment 4. Avantageusement, les poches 10 et 31, sont regroupées en une seule poche à deux chambres complémentaires.
Le volume total représenté alors par les deux chambres 10 et 31 est égal au volume de la chambre 9.

Le procédé de rinçage et d'amorçage selon la présente invention permet d'effectuer, de façon très simple pour le personnel hospitalier, un rinçage efficace des deux compartiments d'un échangeur.

L'utilisation d'une solution stérile pour le rinçage du compartiment dialysat évite d'apporter, lors du rinçage, des substances contaminantes qui seraient éventuellement présentes dans le liquide préparé par le générateur de liquide de dialyse.

En outre, le procédé de rinçage et d'amorçage selon la présente invention peut être effectué alors que l'appareil de dialyse n'est pas encore totalement prêt, c'est-à-dire par exemple pendant la phase de préparation du liquide de dialyse. Ce gain de temps est très important en milieu hospitalier car il permet d'effectuer un plus grand nombre de séances de traitement avec la même quantité d'appareils.

Le procédé de rinçage et d'amorçage selon la présente invention présente également l'avantage pour le personnel hospitalier d'éviter la nécessité des retournements successifs de l'échangeur, tels qu'ils doivent être pratiqués avec les méthodes de l'art antérieur si l'on veut effectuer correctement le dégazage des deux compartiments de l'échangeur.

En effet, selon la présente invention, l'échangeur est dans la meilleure position pour être rincé et amorcé sans introduire de bulles d'air. Les deux compartiments sont remplis de liquide stérile de bas en haut, ce qui est préférable pour ne pas introduire d'air ; l'avantage particulier de la présente invention est que cela peut être obtenu sans retournement ni manipulation de l'échangeur.

En outre, le fait que le liquide circule à co-courant dans les deux compartiments est particulièrement favorable pour maintenir une différence de pression sensiblement constante tout au long de la membrane ; ceci maintient un flux de convexion du compartiment sang vers le compartiment dialysat et évite ainsi les problèmes éventuels de rétrofiltration.

Le procédé selon la présente invention peut être appliqué quelle que soit la forme de la membrane (plane, à fibres creuses, tubulaire aplatie) et quelle que soit sa nature chimique.
Cependant, les membranes présentant une très grande affinité pour les protéines, telles les membranes à base de polyacrylonitrile conviennent particulièrement pour la mise en oeuvre de ce procédé.

## Revendications

1. Procédé de rinçage et d'amorçage d'un échangeur (1) du type comprenant une membrane (2) semi-perméable à deux faces, la première face délimitant un premier compartiment (3) destiné à la circulation du sang à traiter, la seconde face délimitant un second compartiment (4) destiné à la circulation d'un liquide épurateur préparé par un générateur de liquide de dialyse, procédé selon lequel on fait circuler dans le premier compartiment (3) une solution stérile, et on fait circuler dans le second compartiment (4) une solution stérile, caractérisé en ce que :
- on fait circuler la solution stérile dans le premier compartiment (3) et la solution stérile dans le second compartiment (4) à co-courant,
- on effectue un dépôt de protéines sur au moins une face de ladite membrane semi-perméable (2).

2. Procédé selon la revendication 1, caractérisé en ce qu'on effectue ledit dépôt de protéines en faisant circuler dans le premier compartiment (3) de l'hémodialyseur (1) une solution stérile d'albumines.

3. Procédé selon l'une des revendications 1 et 2, caractérisé en ce qu'on effectue ledit dépôt des protéines en faisant circuler dans le second compartiment (4) de l'hémodialyseur (1) une solution stérile d'albumines.

4. Procédé selon la revendication 1, caractérisé en ce qu'on effectue ledit dépôt de protéines postérieurement au rinçage du premier compartiment (3), en faisant circuler le sang à traiter dans le premier compartiment (3), et en maintenant la solution stérile présente dans le second compartiment (4) pendant la première phase de circulation du sang.

5. Procédé selon l'une des revendications précédentes, caractérisé en ce qu'on utilise une source (9,11) de liquide stérile propre à chacun des compartiments (3,4) de l'hémodialyseur (1).

6. Procédé selon l'une des revendications 1 à 4, caractérisé en ce qu'on fait circuler ladite solution stérile successivement dans le premier (3) puis dans le second (4) compartiment de l'hémodialyseur (1).

7. Procédé selon la revendication 6, caractérisé en ce qu'on élimine directement une première fraction de la solution stérile après son passage dans le premier (3) compartiment de l'hémodialyseur.

8. Procédé selon la revendication 7, caractérisé en ce que la première fraction de la solution stérile représente environ 500 ml.

9. Procédé selon les revendications 1, 4, 6, 7 ou 8, caractérisé en ce que ladite solution stérile est une solution saline physiologique héparinée.

## Claims

1. A method for the rinsing and priming of an exchanger (1) of the type comprising a two-sided semipermeable membrane (2), the first side delimiting a first compartment (3) intended for the circulation of the blood to be treated, the second side delimiting a second compartment (4) intended for the circulation of a purifying liquid prepared by a generator of the dialysis liquid, according to which method a sterile solution is caused to circulate in the first compartment (3), and a sterile solution is caused to circulate in the second compartment (4), characterized in that:
- the sterile solution is caused to circulate in the first compartment (3) and the sterile solution in the second compartment (4) in a concurrent flow,
- a deposit of proteins is effected on at least one side of the said semipermeable membrane (2).

2. A method according to claim 1, characterized in that the said deposit of proteins is effected by causing a sterile albumin solution to circulate in the first compartment (3) of the haemodialyser (1)

3. A method according to one of claims 1 and 2, characterized in that the said deposit of proteins is effected by causing a sterile albumin solution to circulate in the second compartment (4) of the haemodialyser (1).

4. A method according to claim 1, characterized in that the said deposit of proteins is effected subsequent to the rinsing of the first compartment (3) by causing the blood to be treated to circulate in the first compartment (3) and by maintaining the sterile solution present in the second compartment (4) during the first stage of the circulation of the blood.

5. A method according to one of the preceding claims, characterized in that a source (9, 11) of the sterile liquid is used which is proper to each one of the compartments (3, 4) of the haemodialyser (1).

6. A method according to one of claims 1 to 4, characterized in that the said sterile solution is caused to circulate successively in the first compartment (3), then in the second compartment (4) of the haemodialyser (1).

7. A method according to claim 6, characterized in that a first fraction of the sterile solution is directly eliminated after passing in the first compartment (3) of the haemodialyser.

8. A method according to claim 7, characterized in that the first fraction of the sterile solution represents approximately 500ml.

9. A method according to claims 1, 4, 6, 7 or 8, characterized in that the said sterile solution is a physiological heparinized saline solution.

## Patentansprüche

1. Verfahren zum Spülen und Inbetriebsetzen einer Austauschvorrichtung (1) der Bauart mit einer zwei Seiten aufweisenden semipermeablen Membran (2), wobei die erste Seite ein erstes Abteil (3) begrenzt, das der Zirkulation des zu behandelnden Bluts dient, während die Zweite Seite ein zweites Abteil (4) begrenzt, das der Zirkulation einer durch einen Dialyseflüssigkeitsgenerator bereiteten Reinigungsflüssigkeit dient, nach welchem Verfahren man im ersten Abteil (3) eine sterile Lösung zirkulieren läßt und im zweiten Abteil (4) eine sterile Lösung zirkulieren läßt,
dadurch **gekennzeichnet,** daß
- man die sterile Lösung im ersten Abteil (3) und die sterile Lösung im zweiten Abteil (4) im Gleichstrom zirkulieren läßt,
- man auf wenigstens einer Seite der semipermeablen Membran (2) einen Niederschlag von Proteinen herbeiführt.

2. Verfahren nach Anspruch 1,
dadurch gekennzeichnet, daß man den Niederschlag von Proteinen dadurch herbeiführt, daß man im ersten Abteil (3) des Blutdialysators (1) eine sterile Lösung von Albuminen zirkulieren läßt.

3. Verfahren nach einem der Ansprüche 1 und 2,
dadurch gekennzeichnet,
daß man den Niederschlag von Proteinen dadurch herbeiführt, daß man im zweiten Abteil (4) des Blutdialysators (1) eine sterile Lösung von Albuminen zirkulieren läßt.

4. Verfahren nach Anspruch 1,
dadurch gekennzeichnet,
daß man den Niederschlag von Proteinen nach dem Spülen des ersten Abteils (3) dadurch herbeiführt, daß man das zu behandelnde Blut im ersten Abteil (3) zirkulieren läßt und daß man während der ersten Zirkulationsphase des Bluts die sterile Lösung im zweiten Abteil (4) anwesend hält.

5. Verfahren nach einem der vorhergehenden Ansprüche,
dadurch gekennzeichnet,
daß man für jedes der Abteile (3, 4) des Blutdialysators (1) eine eigene Quelle (9, 11) für sterile Flüssigkeit verwendet.

6. Verfahren nach einem der Ansprüche 1 bis 4,
dadurch gekennzeichnet,
daß man die sterile Lösung aufeinanderfolgend im ersten (3) und dann im zweiten (4) Abteil des Blutdialysators (1) zirkulieren läßt.

7. Verfahren nach Anspruch 6,
dadurch gekennzeichnet,
daß man unmittelbar nach ihrem Durchgang durch das erste Abteil (3) des Blutdialysators eine erste Fraktion der sterilen Lösung beseitigt.

8. Verfahren nach Anspruch 7,
dadurch gekennzeichnet,
daß die erste Fraktion der sterilen Lösung ungefähr 500 ml beträgt.

9. Verfahren nach den Ansprüchen 1, 4, 6, 7 oder 8,
dadurch gekennzeichnet,
daß die sterile Lösung eine heparinierte physiologische Salzlösung ist.
